# EUROPEAN PATENT SPECIFICATION

(11) **EP 2 190 391 B1**
(45) Date of publication and mention of the grant of the patent: **08.02.2012**
(21) Application number: 08779524.1
(22) Date of filing: 03.07.2008
(51) Int. Cl.: A61F 11/10

(54) **AN INFLATABLE EAR PLUG**
AUFBLASBARER OHRPFROPF
BOUCHON D'OREILLE GONFLABLE

(30) Priority: 26.07.2007 TR 200705205
(43) Date of publication of application: 02.06.2010
(73) Proprietor: Kuralkan, Bekir, Istanbul (TR)
(72) Inventor: Kuralkan, Bekir, Istanbul (TR)
(74) Representative: Kaya, Erdem
(86) International application number: PCT/TR2008/000078
(87) International publication number: WO 2009/014505

(56) References cited:
- GB-A- 2 407 274
- US-A- 2 719 523
- US-A- 4 913 165
- US-A1- 2008 144 871

## Description

### Field of Invention

The present invention relates to ear plugs used to avoid the intrusion of water-like foreign substances into the middle ear, and more particularly to inflatable ear plugs which comprise a main body incorporating a liquid reservoir, an pliable piece in connection with said main body, and an inflation mechanism to transfer the liquid in said liquid reservoir to the pliable piece.

### Prior Art

The present invention more specifically relates to inflatable ear plugs, which becomes inflated once a medical liquid which is not harmful to human health is forced by rotating the accurate adjustment means so that the pliable-material element in the part that settles into the ear assumes the shape of the user's ear, both providing efficient sealing, and not becoming easily dislocated from its position in the ear while the user is under action, since it completely settles into the ear, thereby fulfilling the sealing function properly.

An example of an inflatable underwater eardrum protector can be found in US 4913 165.

Ear plugs, as known, are made from materials pliable enough to become settled into the ear once forced therein for avoiding the intrusion of foreign substances into the middle ear. Some injuries, e.g. puncture of eardrums may occur in some people for some reasons. With the intrusion into the middle ear of substances such as water, such injuries may bring about some health-related problems such as otitis media - inflammation of the ear, balance disorders, etc. Such injured ears must, for this reason, be protected from substances to come externally. In brief, ear plugs are produced in order to fulfill this function.

According to an application within the relevant prior art, a specialized physician casts the ear mold in compliance with a suffering person's ears, producing ear plugs which would comply with the ear structure. Since this approach requires a physician's treatment, it is both costly and unpractical. Additionally, since each ear plug under this approach has a distinct shape, it avoids the conduction of mass production.

In order to eliminate this cost problem, for example, the patent document ES2087031 discloses an inflatable type of ear plug, which is produced uniformly, and which assumes the shape of ear once the pliable part of said ear plug is inflated by means of an external and separate device so as to provide the sealing effect. Accordingly, there is used an air-supplying plug mechanism for inflating the pliable part of said ear plug which is inserted into the ear by means of pumping the ambient air. This mechanism transfers the air supplied from the air pump into the ear-settled pliable plug, such that the ear plug becomes inflated and assumes the shape of ear, settling completely to provide an efficient sealing and protect the ear from foreign substances such as water.

This embodiment, however, has some drawbacks. It requires a separate air-supplying device for inflating the plug inserted into the ear. This requirement both avoids the practical use of the ear plug, and makes it more costly as compared to other ear plugs. Furthermore, the ear plug is not inflated very accurately. This inaccuracy has the potential of discomforting the user while the ear plug is inflated.

In result, all drawbacks defined above made it necessary to bring a novelty in the related prior art.

### Brief Description of Invention

The present invention relates to a novel inflatable ear plug as described in claim 1, eliminating all aforesaid problems and brining additional advantages to the relevant prior art.

Accordingly, the main objective of the present invention is to produce an inflatable ear plug comprising an internal inflation mechanism, both capable of providing a more efficient sealing, and being produced more simply, used practically, and being of lower cost as compared to equivalent products.

According to this main objective, another objective of the present invention is to provide an ear plug embodiment of which the inflation rate is controlled and adjusted in an extremely accurate manner.

In order to achieve the objectives referred to hereinabove and to be elaborated hereunder, the present invention provides an inflatable ear plug comprising a main body incorporating a liquid reservoir, a pliable piece in connection with said liquid reservoir, and an inflation mechanism for transferring the liquid in said liquid reservoir to the pliable piece, said inflatable ear plug being characterized in that said inflation mechanism comprises an adjustment piece which is connected to said main body so as to rotate around itself, and a piston arrangement as described in claim 1, controlled by means of said adjustment piece in order to move said liquid.

In a preferred embodiment of the present invention, said adjustment piece has a bullet-like shape and is an adjustment tail in which a part of said piston arrangement is positioned.

Said piston arrangement comprises a tail nut which is capable of rotating together with the adjustment tail and at least of which the internal-facing surface is threaded, and a threaded bar which is in connection with said tail nut and thereby functions as a piston by moving back and forth and comprises a forcing surface on its side that is in contact with the liquid.

In a further preferred embodiment of the present invention, the subject ear plug comprises a surface where a neck strap can be attached to in order to wear said ear plug around the neck.

Still in a further preferred embodiment of the present invention, said surface is a strap protrusion provided on the cover part, said strap protrusion having a form which allows to pass said neck strap there through.

Yet in a further preferred embodiment of the present invention, the ear plug comprises a plug tube which extends downward from the lower part of main body in the interior of the pliable piece for maintaining said pliable piece in a certain form.

In another preferred embodiment of the present invention, the subject ear plug further comprises a cover provided at the other end of main body.

In a further preferred embodiment of the present invention, the subject ear plug has a shape that resembles a hair dryer.

Structural and characteristic features and all advantages of the present invention shall be made clear by means of annexed figures described here below and a detailed description written by making references to said figures; therefore the present invention must be evaluated by taking into consideration these figures and the detailed description.

### Brief Description of Figures

Figure 1 gives a general view of the inflatable ear plug embodiment according to the present invention.
Figure 2 gives a cross-sectional perspective view of the inflatable ear plug embodiment according to the present invention.
Figure 3 illustrates an alternative embodiment of the inflatable ear plug according to the present invention.
Figure 4 illustrates another alternative embodiment of the inflatable ear plug according to the present invention.

### Reference Numbers in Figures

1. Main body
1.1 Liquid reservoir
1.2 Cover
1.3 Body extension
1.4 Level indicators
1.5 Cover hook
2. Pliable piece
2.1 Plug tube
3. Adjustment tail
3.1 Threaded bar
3.2 Forcing surface
3.3 Tail nut
3.4 Tail nut cover
3.5 Felt

### Detailed description of the preferred embodiment

In the following detailed description, the subject inflatable ear plug embodiment shall be described illustratively by making references to annexed figures, only to make it clear without imposing any restrictions thereon.

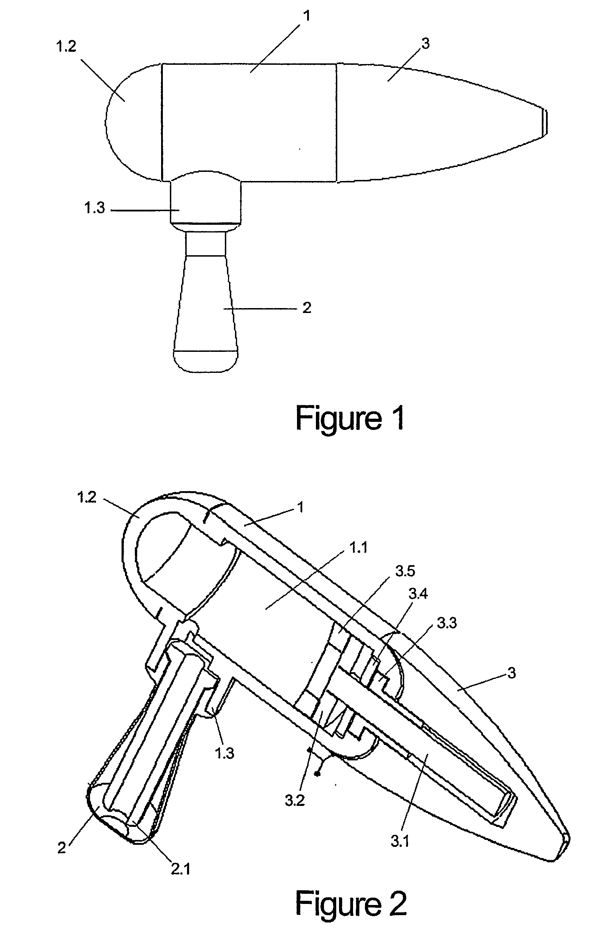

As illustrated in Figure 1, the ear plug according to the present invention comprises in the most general meaning a main body (1) which with its interior volume defines a liquid reservoir (1.1), an adjustment tail (3) connected to one end of said main body (1) so as to rotate around itself, a cover (1.2) positioned at the other end of said main body (1), a body extension (1.3) extending downwards from the lower part of main body (1), and a pliable piece (2) attached to said body extension (1.3). As can be seen in this Figure as well, this embodiment resembles the general shape of hair dryers. On the other hand, said pliable piece (2) is produced preferably from silicone, and the remaining parts illustrated in Figure 1 preferably from a plastic-based material.

As illustrated in Figure 2, the pliable piece (2) is connected to the main body (1) that accommodates a liquid reservoir (1.1) by means of the body extension (1.3). A liquid, (1.4), not harmful to human health such as distilled water, is filled through the cover (1.2) part provided on the main body (1) during manufacture. As again illustrated in this Figure, the inflation mechanism (not illustrated in figures) is positioned so that it is partially left in the adjustment tail (3) and partially left in the liquid reservoir (1.4). This mechanism comprises in the most general sense a tail nut (3.3) which can move together with the adjustment tail (3) and has a threaded surface, and a threaded bar (3.1) engaged into said tail nut (3.3). The tail nut (3.3) is fixed by means of a tail nut cover (3.4) provided at a point where the main body (1) unifies with the adjustment tail (3). As for the threaded bar (3.1), it comprises a felt (3.5) and a forcing surface (3.2) at its end facing the liquid reservoir (1.1).

According to the structural details given above, the operation of the embodiment according to the present invention is as follows. When the user wishes to use the ear plug according to the present invention he/she rotates the adjustment tail (3); the rotation of this adjustment tail (3) causes the rotation of the tail nut (3.3), driving the threaded bar (3.1) forward, and forcing the liquid in the liquid reservoir (1.1) towards the pliable piece (2) by means of the forcing surface (3.2) comprised by said threaded bar (3.1). Therefore the liquid is forced to pass through the plug tube (2.1) under the effect of this force and starts to fill and inflate the pliable piece (2). The pliable piece (2) gradually assumes the shape of the user's ear as a result of filling, whereas the inflation operation is carried on until the ear plug does not dislocate from the ear and provides an efficient sealing. In alternative embodiments of the present invention, said medical liquid (1.4) may be a low-cost liquid (1.4), such as water that would not harm human health.

In result, and as differing from the equivalent embodiments of the prior art, the ear plug (2) according to the present invention is capable to inflate and deflate by itself by means of its own mechanism without requiring any external support, thanks to the structure of the adjustment tail (3) and the liquid (1.4). Additionally, the user of the ear plug has the opportunity to easily use the ear plug whenever he/she desires, thanks to inflating and deflating the ear plug according to the present invention (2) in an easy manner whenever he/she desires to do so. Furthermore, the ear plug according to the present invention can be produced in a standard, low-cost, and swift manner, as it becomes inflated by itself to assume the shape of the user's ear.

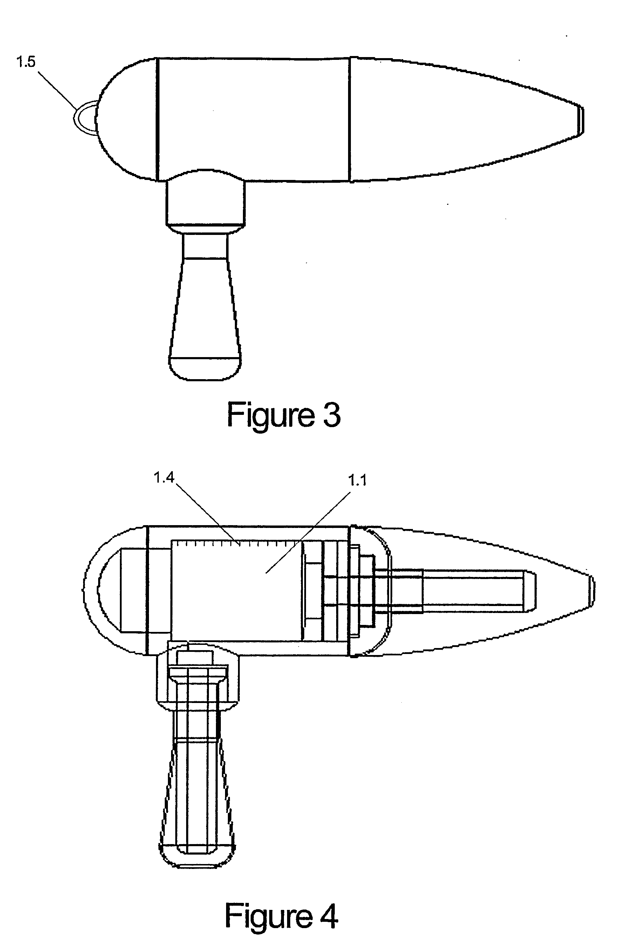

As illustrated in Figure 3, an alternative of the present invention can be structured so as to allow the user to wear said ear plug around his/her neck and carry/use it this way, by passing a means which is made from string-, thread-like material to be worn around the neck through a hook protrusion (1.5) to be embodied at the cover (1.2) part.

In another alternative embodiment of the present invention, the ear plug according to the present invention comprises visual elements reminding the ideal inflation rate for the user. In one embodiment, for instance, the main body (1) is made transparently with various level indicators thereon to visualize the liquid (1.4) and the displacement extent of the piston therein.

The shape and materials to be used in alternative embodiments and parts of the present invention may of course be replaced with others that would not harm human health. In result, the protection scope of the present invention is set forth in appended Claims and cannot be restricted to the illustrative disclosures given above, under the detailed description. It is because a person skilled in the relevant art can obviously produce similar embodiments under the light of the foregoing disclosures, without departing from the main principles of the present invention.

## Claims

1. An inflatable ear plug comprising a main body (1) incorporating a liquid reservoir (1.2), a pliable piece (2) in connection with said liquid reservoir (1.2), and an inflation mechanism for transferring the liquid (1.4) in said liquid reservoir (1.2) to the pliable piece (2), said inflation mechanism having an adjustment piece (3) which is connected to said main body (1) so as to rotate around itself, and a piston arrangement controlled by means of said adjustment piece (3) in order to move said liquid (1.4) **characterized in that** said piston arrangement comprises a tail nut (3.3) which is capable of rotating together with the adjustment tail (3) and at least of which the internal-facing surface is threaded, and a threaded bar (3.1) which is in connection with the threaded surface of said tail nut (3.3) and thereby functions as a piston by moving back and forth and comprises a forcing surface (3.2) on its side that is in contact with the liquid (1.4).

2. An ear plug according to Claim 1, **characterized in that** said adjustment piece (3) has a bullet-like shape and is an adjustment tail (3) wherein a part of said piston arrangement is positioned.

3. An ear plug according to any of the preceding claims, **characterized by** comprising a surface whereto a neck strap can be attached in order to wear said ear plug around the neck.

4. An ear plug according to Claim 3, **characterized in that** said surface is a strap protrusion provided on the cover (1.2) part, said strap protrusion having a form which allows to pass said neck strap there through.

5. An ear plug according to any of the preceding claims, **characterized by** further comprising a plug tube (2.1) which extends downward from the lower part of main body (1) in the interior of the pliable piece (2) for maintaining said pliable piece (2) in a certain form.

6. An ear plug according to any of the preceding claims, **characterized by** further comprising a cover (1.2) positioned on the other end of said main body (1).

7. An ear plug according to any of the preceding claims, **characterized in that** said ear plug has a shape that resembles a hair dryer.

## Patentansprüche

1. Aufblasbarer Ohrpfropf, umfassend einen Hauptkörper (1), der einen Flüssigkeitsbehälter (1.2), ein biegsames Teil (2) in Verbindung mit dem Flüssigkeitsbehälter (1.2) und einen Aufblasmechanismus zum Übertragen der Flüssigkeit (1.4) in dem Flüssigkeitsbehälter (1.2) zu dem biegsamen Teil (2) enthält, wobei der Aufblasmechanismus ein Einstellteil (3) aufweist, das mit dem Hauptkörper (1) verbunden ist, sodass es sich um sich selbst dreht, und eine Kolbenanordnung, die mittels des Einstellteils (3) gesteuert wird, um die Flüssigkeit (1.4) zu bewegen, **dadurch gekennzeichnet, dass** die Kolbenanordung eine Endstückmutter (3.3) umfasst, die sich gemelnsam mit dem Einstellendstuck (3) drehen kann und von der mindestens die nach innen zeigende Fläche mit einem Gewinde versehen ist, und eine Gewindestange (3.1), die mit der Gewindefläche der Endstückmutter (3.3) in Verbindung steht und dadurch durch Hin- und Herbewegen als Kolben funktioniert und eine Druckfläche (3.2) an der Seite umfasst, die die Flüssigkeit (1.4) berührt.

2. Ohrpfropf nach Anspruch 1, **dadurch gekennzeichnet, dass** das Einstellteil (3) eine geschossähnliche Form aufweist und ein Einstellendstück (3) ist, in dem ein Teil der Kolbenanordnung platziert ist.

3. Ohrpfropf nach einem der vorhergehenden Ansprüche, **dadurch gekennzeichnet, dass** er eine Fläche umfasst, an der ein Trageriemen befestigt werden kann, um den Ohrpfropf um den Hals zu tragen.

4. Ohrpfropf nach Anspruch 3, **dadurch gekennzeichnet, dass** die Fläche ein Riemenvorsprung ist, der an dem Abdeckungsteil (1.2) vorgesehen ist, wobei der Riemenvorsprung eine Form aufweist, die es ermöglicht, dass der Trageriemen hindurchgeführt wird.

5. Ohrpfropf nach einem der vorhergehenden Ansprüche, **dadurch gekennzeichnet, dass** er ferner eine Pfropfröhre (2.1) umfasst, die sich vom unteren Teil des Hauptkörpers (1) aus im Inneren des biegsamen Teils (2) nach unten erstreckt, um das biegsame Teil (2) in einer bestimmten Form zu halten.

6. Ohrpfropf nach einem der vorhergehenden Ansprüche, **dadurch gekennzeichnet, dass** er ferner eine Abdeckung (1.2) umfasst, die am anderen Ende des Hauptkörpers (1) platziert ist.

7. Ohrpfropf nach einem der vorhergehenden Ansprüche, **dadurch gekennzeichnet, dass** der Ohrpfropf eine Form aufweist, die einem Haartrockner ähnelt.

## Revendications

1. Bouchon d'oreille gonflable comprenant un corps principal (1) comprenant un réservoir de liquide (1.2), une pièce pliable (2) conjointement avec ledit réservoir de liquide (1.2) et un mécanisme de gonflage pour transférer le liquide (1.4) dans ledit réservoir de liquide (1.2) à la pièce pliable (2), ledit mécanisme de gonflage ayant une pièce d'ajustement (3) qui est raccordée audit corps principal (1) afin de tourner autour d'elle-méme, et un agencement de piston commandé au moyen de ladite pièce d'ajustement (3) afin de déplacer ledit liquide (1.4), **caractérisé en ce que** ledit agencement de piston comprend un écrou à queue (3.3) qui peut tourneur conjointement avec la queue d'ajustement (3) et dont au moins la surface orientée vers l'intérieur est filetée, et une barre filetée (3.1) qui est en rapport avec la surface filetée dudit écrou à queue (3.3) et fonctionne ainsi comme un piston en se déplaçant en avant et en arrière et comprend une surface de pression (3.2) sur son côté qui est en contact avec le liquide (1.4).

2. Bouchon d'oreille selon la revendication 1, **caractérisé en ce que** ladite pièce d'ajustement (3) a une forme de balle et est une queue d'ajustement (3) dans laquelle une partie dudit agencement de piston est positionnée.

3. Bouchon d'oreille selon l'une quelconque des revendications précédentes, **caractérisé en ce qu'**il comprend une surface où une sangle de cou peut être fixée afin de porter ledit bouchon d'oreille autour du cou.

4. Bouchon d'oreille selon la revendication 3, **caractérisé en ce que** ladite surface est une saillie de sangle prévue sur la partie de couvercle (1.2), ladite saillie de sangle ayant une forme qui permet à ladite sangle de cou de passeur à travers cette dernière.

5. Bouchon d'oreille selon l'une quelconque des revendications précédentes, **caractérisé en ce qu'**il comprend en outre un tube de bouchon (2.1) qui s'étend vers le bas à partir de la partie inférieure du corps principal (1) dans l'intérieur de la pièce pliable (2) pour maintenir ladite pièce pliable (2) dans une certaine forme.

6. Bouchon d'oreille selon l'une quelconque des revendications précédente, **caractérisé en ce qu'**il comprend en outre un couvercle (1.2) positionné sur l'autre extrémité dudit corps principal (1).

7. Bouchon d'oreille selon l'une quelconque des revendications précédentes, **caractérisé en ce que** ledit bouchon d'oreille a une forme qui ressemble à un sèche-cheveux,
